# EUROPEAN PATENT APPLICATION

(11) **EP 1 553 087 A1**
(43) Date of publication of application: **13.07.2005**
(21) Application number: 03794292.7
(22) Date of filing: 09.09.2003
(51) Int. Cl.: C07D 231/20, A61K 31/4152, A61P 1/00, A61P 43/00

(54) **PREVENTIVE AND/OR THERAPEUTIC DRUGS FOR INFLAMMATORY INTESTINAL DISEASES**

(30) Priority: 09.09.2002 JP 2002262324
(71) Applicant: Mitsubishi Pharma Corporation, Osaka-shi, Osaka 541-0046 (JP)
(72) Inventor: ARAKI, Yoshio, Ootsu-shi, Shiga 520-2101 (JP); ANDOH, Akira, Kusatsu-shi, Shiga 525-0045 (JP); FUJIYAMA, Yoshihide, Ootsu-shi, Shiga 520-0865 (JP)
(74) Representative: ter Meer, Nicolaus, Dipl.-Chem., Dr.
(86) International application number: PCT/JP2003/011487
(87) International publication number: WO 2004/022543

(57) **Abstract**

The object of the present invention is to provide a medicament useful for preventing and/or treating inflammatory bowel disease. The present invention provides a medicament for preventing and/or treating inflammatory bowel disease which comprises as an active ingredient a pyrazolone derivative represented by the following formula (I) or a physiologically acceptable salt thereof, or a hydrate thereof or a solvate thereof: 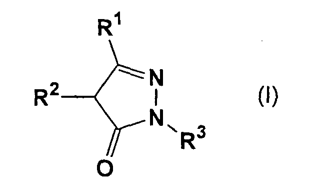 wherein R¹ represents a hydrogen atom, an aryl group, an alkyl group, or an alkoxycarbonylalkyl group; R² represents a hydrogen atom, an aryloxy group, an arylmercapto group, an alkyl group or a hydroxyalkyl group; or R¹ and R² are combined with each other to represent an alkylene group; and R³ represents a hydrogen atom, an alkyl group, a cycloalkyl group, a hydroxyalkyl group, a benzyl group, a naphthyl group, a phenyl group, or a phenyl group substituted with 1 to 3 substituents selected from the group consisting of an alkyl group, an alkoxy group, a hydroxyalkyl group, an alkoxycarbonyl group, an alkylmercapto group, an alkylamino group, a dialkylamino group, a halogen atom, a trifluoromethyl group, a carboxyl group, a cyano group, a hydroxyl group, a nitro group, an amino group and an acetamide group.

## Description

### Technical Field

The present invention relates to a medicament for preventing and/or treating inflammatory bowel disease, an intestinal mucosa protective agent, an agent for inhibiting the activation of neutrophilic leucocytes, and an agent for inhibiting myeloperoxidase activity, all of which comprise, as an active ingredient, a pyrazolone derivative or a physiologically acceptable salt thereof, or a hydrate thereof or a solvate thereof.

### Background Art

Various causative factors have been considered regarding the development of inflammatory bowel disease (IBD), such as ulcerative colitis (UC) or Crohn's disease (CD). Such possible causative factors may include bacterial infection and autoimmune disorder. However, at present, the cause is still unknown. Since the incidence rate of the above disease has increased along with the development of civilization, it is suggested that environmental factors such as stress be involved in the disease. In order to treat the disease, when the disease is ulcerative colitis, at present, main treatments may include the oral administration of an agent comprising, as an active ingredient, salazosulfapyridine or aminosalicylic acid such as 5-aminosalicylic acid, and the oral, enema or suppository administration of adrenocorticosteroid (predonisolone). In addition, immunosuppressive agents such as azathioprine or 6-mercaptopurine (6-MP) may also be administered at time. On the other hand, when the disease is Crohn's disease, nutritional treatment is mainly applied (elemental diet and central venous nutrition), and additionally, adrenocortical hormone or salazosulfapyridine may also orally be administered. However, in both cases of ulcerative colitis and Crohn's disease, refractory symptoms have been reported, and the side effects caused by the therapeutic agents are also problematic. Surgical treatments are selected for such intractable or severe cases under the present circumstances.

Dextran sulfate sodium-induced colitis (hereinafter referred to as DSS-induced colitis) is frequently used as a human ulcerative colitis model for the studies regarding colitis development mechanism or for the development of therapeutic agents for colitis. The mechanism whereby DSS-induced colitis is developed has not been clarified, but it is considered that it is mainly caused by the toxicity of dextran sulfate sodium per se. It has recently been found that free radicals play an important role in the progression of DSS-induced colitis. For example, Dykens et al. have reported that free radicals derived from infiltrating leucocyte myeloperoxidase (MPO) are associated with the progression of DSS-induced colitis (Dykens J. A. et al., Scand J Gastroenterol, 33, 628-636, 1998). In addition, Hori (Hori Y. et al., Jpn J Pharmacol, 74, 99-103, 1997) and Naito (Naito Y. et al., Antioxid Redox Signal, 4, 195-206, 2002) have found that superoxide dismutase (SOD) and a spin-trapping agent have therapeutic effects on DSS-induced colitis, respectively. They have also reported that free radicals are associated with the progression of DSS-induced colitis. Moreover, Blackburn et al. have demonstrated the generation of free radicals in DSS-induced colitis by high performance liquid chromatography (Blackburn A. C. et al., Free Radic Biol Med, 25, 305-313, 1998). Furthermore, Korenaga et al. have found that in DSS-induced colitis, not only free radicals are generated, but also the resistivity of the intestinal mucosa to free radicals decreases (Korenaga D. et al., J Surg Res, 144-149, 102, 2002).

Regarding a pyrazolone derivative which is represented by the following formula (I): 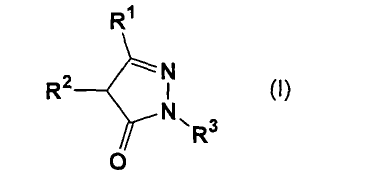 wherein R¹ represents a hydrogen atom, aryl, C₁₋₅ alkyl, or C₃₋₆ (total carbon number) alkoxycarbonylalkyl, R² represents a hydrogen atom, aryloxy, arylmercapto, C₁₋₅ alkyl or C₁₋₃ hydroxyalkyl, or R¹ and R² are combined with each other to represent C₃₋₅ alkylene group, and R³ represents a hydrogen atom, C₁₋₅ alkyl, C₅₋₇ cycloalkyl, C₁₋₃ hydroxyalkyl, benzyl, naphthyl or phenyl, or phenyl substituted with the same or different 1 to 3 substituents selected from the group consisting of C₁₋₅ alkoxy, C₁₋₃ hydroxyalkyl, C₂₋₅ (total carbon number) alkoxycarbonyl, C₁₋₃ alkylmercapto, C₁₋₄ alkylamino, C₂₋₈ (total carbon number) dialkylamino, halogen atom, trifluoromethyl, carboxyl, cyano, hydroxyl group, nitro, amino and acetamide), examples of the known medical applications include cerebral function-normalizing action (JP Patent Publication (Kokoku) No. 5-31523 B (1993)), lipid peroxide production-suppressing action (JP Patent Publication (Kokoku) No. 5-35128, B (1993), antiulcer action (JP Patent Publication (Kokai) No. 3-215425 (1991)) and anti-hyperglycemic action (JP Patent Publication (Kokai) No. 3-215426 (1991)).

Among the compounds represented by the above formula (I), a pharmaceutical preparation containing 3-methyl-1-phenyl-2-pyrazolin-5-one as an active ingredient has been commercially available as a protective agent for the brain (under the general name "edaravone" and the commercial name "Radicut": produced and marketed by Mitsubishi Pharma Corporation) since June 2001. This "edaravone" has been reported to have high reactivity to active oxygen (Kawai, H., et al., J. Phamacol. Exp. Ther., 281(2), 921, 1997; Wu, TW. et al., Life Sci, 67(19), 2387, 2000). As described above, edaravone is a free radical scavenger which prevents cell damage and the like by removing various free radicals including active oxygen.

As stated above, it is suggested that superoxide anions derived from macrophage or leucocytes or free radicals such as hydroxy radicals or hydrogen peroxide are associated with the progression of ulcerative colitis. However, there have been no reports on the studies regarding the effectiveness of edaravone for inflammatory bowel disease.

### Disclosure of the Invention

It is an object of the present invention to provide a medicament useful for preventing and/or treating inflammatory bowel disease, an intestinal mucosa protective agent, an agent for inhibiting the activation of neutrophilic leucocytes, and an agent for inhibiting myeloperoxidase activity.

As a result of intensive studies directed towards achieving the aforementioned object, the present inventors have found that a pyrazolone derivative represented by formula (I) or a physiologically acceptable salt thereof, or a hydrate thereof or a solvate thereof, significantly reduces the mucosa area injured by colitis or the wet weight of the large intestine that indicates the degree of edema in a dextran sulfate sodium-induced colitis model, and that the above derivative and the like has strong action to inhibit colitis. The present invention has been completed based on the aforementioned findings.

According to the present invention, there is provided a medicament for preventing and/or treating inflammatory bowel disease which comprises as an active ingredient a pyrazolone derivative represented by the following formula (I) or a physiologically acceptable salt thereof, or a hydrate thereof or a solvate thereof: 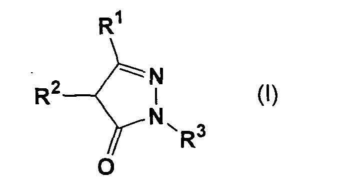 wherein R¹ represents a hydrogen atom, an aryl group, a C₁₋₅ alkyl group, or a C₃₋₆ (total carbon number) alkoxycarbonylalkyl group; R² represents a hydrogen atom, an aryloxy group, an arylmercapto group, a C₁₋₅ alkyl group or a C₁₋₃ hydroxyalkyl group; or R¹ and R² are combined with each other to represent C₃₋₅ alkylene group; and R³ represents a hydrogen atom, a C₁₋₅ alkyl group, a C₅₋₇ cycloalkyl group, a C₁₋₃ hydroxyalkyl group, a benzyl group, a naphthyl group, a phenyl group, or a phenyl group substituted with the same or different 1 to 3 substituents selected from the group consisting of a C₁₋₅ alkyl group, a C₁₋₅ alkoxy group, a C₁₋₃ hydroxyalkyl group, a C₂₋₅ (total carbon number) alkoxycarbonyl group, a C₁₋₃ alkylmercapto group, a C₁₋₄ alkylamino group, a C₂₋₈ (total carbon number) dialkylamino group, a halogen atom, a trifluoromethyl group, a carboxyl group, a cyano group, a hydroxyl group, a nitro group, an amino group and an acetamide group.

According to a preferred embodiment of the present invention, there is provided the aforementioned medicament wherein the pyrazolone derivative represented by the formula (I) is 3-methyl-1-phenyl-2-pyrazolin-5-one or a physiologically acceptable salt thereof, or a hydrate thereof or a solvate thereof.

In another aspect, the present invention provides an intestinal mucosa protective agent, an agent for inhibiting the activation of neutrophilic leucocytes, and an agent for inhibiting myeloperoxidase activity, all of which comprise, as an active ingredient, a pyrazolone derivative represented by formula (I) or a physiologically acceptable salt thereof, or a hydrate thereof or a solvate thereof.

In another aspect, the present invention provides a method for preventing and/or treating inflammatory bowel disease, a method for protecting the intestinal mucosa, a method for inhibiting the activation of neutrophilic leucocytes, and a method for inhibiting myeloperoxidase activity, all of which comprise a step of administering to mammals such as a human, a pyrazolone derivative represented by the formula (I) or a physiologically acceptable salt thereof, or a hydrate thereof or a solvate thereof, at an amount that is effective for prevention and/or treatment of the above-described disease.

In another aspect, the present invention provides the use of a pyrazolone derivative represented by formula (I) or a physiologically acceptable salt thereof, or a hydrate thereof or a solvate thereof, for the production of a medicament for preventing and/or treating inflammatory bowel disease, an intestinal mucosa protective agent, an agent for inhibiting the activation of neutrophilic leucocytes, or an agent for inhibiting myeloperoxidase activity.

### Brief Description of the Drawings

Figure 1(A) shows the wet weight of the large intestine in each administration group. Figure 1(B) shows the injured mucosa area in each administration group.
Figure 2 shows the image of large intestine tissues in each administration group ((A) control group (x 40), (B) 1 mg/kg edaravone administration group (x 40), (C) 5 mg/kg edaravone administration group (x 40), and (D) 20 mg/kg edaravone administration group (x 40)).
Figure 3(A) shows the damage score of each administration group. Figure 3(B) shows myeloperoxidase activity in the large intestinal mucosa in each administration group.
Figure 4(A) shows the concentration of MDA and that of 4-HNE in the large intestinal mucosa in each administration group. Figure 4(B) shows IL-6 in the plasma in each administration group.

### Best Mode for Carrying out the Invention

The medicament for preventing and/or treating inflammatory bowel disease, the intestinal mucosa protective agent, the agent for inhibiting the activation of neutrophilic leucocytes, and the agent for inhibiting myeloperoxidase activity according to the present invention (which are hereinafter generically referred to as "the medicament of the present invention") comprise the pyrazolone derivative represented by the formula (I) as defined in this specification or a physiologically acceptable salt thereof, or a hydrate thereof or a solvate thereof.

The compound represented by the formula (I) used in the present invention can have a structure represented by the following formula (I') or (I") due to tautomerism. One of the tautomers is shown in the formula (I) of this specification for convenience. The presence of the following tautomers is obvious to a person skilled in the art. As an active ingredient of the medicament of the present invention, the compound represented by the following formula (I') or (I") or a physiologically acceptable salt thereof, or a hydrate thereof or a solvate thereof may also be used.

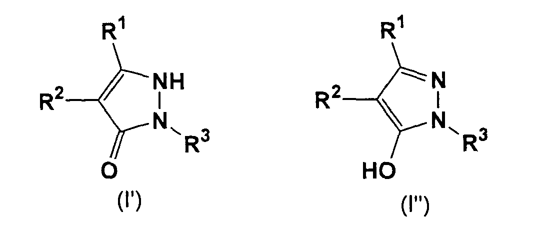

In the formula (I), the aryl group in the definition of R¹ may be either a monocyclic or polycyclic aryl group. Examples thereof include a phenyl group, a naphthyl group and the like, as well as a phenyl group substituted with a substituent such as an alkyl group (for example, a methyl group or a butyl group), an alkoxy group (for example, a methoxy group or a butoxy group), a halogen atom (for example, a chlorine atom) or a hydroxy group. The same applies for aryl portions in other substituents (e.g., an aryloxy group) having the aryl portions.

The C₁₋₅ alkyl group in the definition of R¹, R² and R³ may be either linear- or branched chain. Examples thereof include a methyl group, an ethyl group, a propyl group, an isopropyl group, a butyl group, an isobutyl group, a sec-butyl group, a tert-butyl group, and a pentyl group. The same applies for alkyl portions in other substituents (alkoxycarbonylalkyl group) having the alkyl portions.

Examples of the C₃₋₆ (total carbon number) alkoxycarbonylalkyl group in the definition of R¹ include a methoxycarbonylmethyl group, an ethoxycarbonylmethyl group, a propoxycarbonylmethyl group, a methoxycarbonylethyl group and a methoxycarbonylpropyl group.

Examples of the aryloxy group in the definition of R² include a p-methylphenoxy group, a p-methoxyphenoxy group, a p-chlorophenoxy group and a p-hydroxyphenoxy group. Examples of the arylmercapto group include a phenylmercapto group, a p-methylphenylmercapto group, a p-methoxyphenylmercapto group, a p-chlorophenylmercapto group and a p-hydroxyphenylmercapto group.

Examples of the C₁₋₃ hydroxyalkyl group in the definition of R² and R³ include a hydroxymethyl group, a 2-hydroxyethyl group and a 3-hydroxypropyl group. Examples of the C₅₋₇ cycloalkyl group in the definition of R³ include a cyclopentyl group, a cyclohexyl group and a cycloheptyl group.

In the definition of R³, examples of the C₁₋₅ alkoxy group that is the substituent of a phenyl group include a methoxy group, an ethoxy group, a propoxy group, an isopropoxy group, a butoxy group and a pentyloxy group; examples of the C₂₋₅ (total carbon number) alkoxycarbonyl group include a methoxycarbonyl group, an ethoxycarbonyl group, a propoxycarbonyl group and a butoxycarbonyl group; examples of the C₁₋₃ alkylmercapto group include a methylmercapto group, an ethylmercapto group and a propylmercapto group; examples of the C₁₋₄ alkylamino group include a methylamino group, an ethylamino group, a propylamino group and a butylamino group; and examples of the C₂₋₈ (total carbon number) dialkylamino group include a dimethylamino group, a diethylamino group, a dipropylamino group, and a dibutylamino group.

Examples of the compound (I) that is preferably used as an active ingredient of the medicament of the present invention include the following compounds.
3-methyl-1-phenyl-2-pyrazolin-5-one;
3-methyl-1-(2-methylphenyl)-2-pyrazolin-5-one;
3-methyl-1-(3-methylphenyl)-2-pyrazolin-5-one;
3-methyl-1-(4-methylphenyl)-2-pyrazolin-5-one;
3-methyl-1-(3,4-dimethylphenyl)-2-pyrazolin-5-one;
1-(4-ethylphenyl)-3-methyl-2-pyrazolin-5-one;
3-methyl-1-(4-propylphenyl)-2-pyrazolin-5-one;
1-(4-butylphenyl)-3-methyl-2-pyrazolin-5-one;
1-(3-trifluoromethylphenyl)-3-methyl-2-pyrazolin-5-one;
1-(4-trifluoromethylphenyl)-3-methyl-2-pyrazolin-5- one;
1-(2-methoxyphenyl)-3-methyl-2-pyrazolin-5-one;
1-(3-methoxyphenyl)-3-methyl-2-pyrazolin-5-one;
1-(4-methoxyphenyl)-3-methyl-2-pyrazolin-5-one;
1-(3,4-dimethoxyphenyl)-3-methyl-2-pyrazolin-5-one;
1-(4-ethoxyphenyl)-3-methyl-2-pyrazolin-5-one;
3-methyl-1-(4-propoxyphenyl)-2-pyrazolin-5-one;
1-(4-butoxyphenyl)-3-methyl-2-pyrazolin-5-one;
1-(2-chlorophenyl)-3-methyl-2-pyrazolin-5-one;
1-(3-chlorophenyl)-3-methyl-2-pyrazolin-5-one;
1-(4-chlorophenyl)-3-methyl-2-pyrazolin-5-one;
1-(3,4-dichlorophenyl)-3-methyl-2-pyrazolin-5-one;
1-(4-bromophenyl)-3-methyl-2-pyrazolin-5-one;
1-(4-fluorophenyl)-3-methyl-2-pyrazolin-5-one;
1-(3-chloro-4-methylphenyl)-3-methyl-2-pyrazolin-5-one;
1-(3-methylmercaptophenyl)-3-methyl-2-pyrazolin-5-one;
1-(4-methylmercaptophenyl)-3-methyl-2-pyrazolin-5-one;
4-(3-methyl-5-oxo-2-pyrazoline-1-yl) benzoic acid;
1-(4-ethoxycarbonylphenyl)-3-methyl-2-pyrazolin-5-one;
1-(4-nitrophenyl)-3-methyl-2-pyrazolin-5-one;
3-ethyl-1-phenyl-2-pyrazolin-5-one;
1-phenyl-3-propyl-2-pyrazolin-5-one;
1,3-diphenyl-2-pyrazolin-5-one;
3-phenyl-1-(p-tolyl)-2-pyrazolin-5-one;
1-(4-methoxyphenyl)-3-phenyl-2-pyrazolin-5-one;
1-(4-chlorophenyl)-3-phenyl-2-pyrazolin-5-one;
3,4-dimethyl-1-phenyl-2-pyrazolin-5-one;
4-isobutyl-3-methyl-1-phenyl-2-pyrazolin-5-one;
4-(2-hydroxyethyl)-3-methyl-1-phenyl-2-pyrazolin-5-one;
3-methyl-4-phenoxy-1-phenyl-2-pyrazolin-5-one;
3-methyl-4-phenylmercapto-1-phenyl-2-pyrazolin-5-one;
3,3 ', 4,5,6,7-hexahydro-2-phenyl-2H-indazole-3-one;
3-(ethoxycarbonylmethyl)-1-phenyl-2-pyrazolin-5-one;
1-phenyl-2-pyrazolin-5-one;
3-methyl-2-pyrazolin-5-one;
1,3-dimethyl-2-pyrazolin-5-one;
1-ethyl-3-methyl-2-pyrazolin-5-one;
1-butyl-3-methyl-2-pyrazolin-5-one;
1-(2-hydroxyethyl)-3-methyl-2-pyrazolin-5-one;
1-cyclohexyl-3-methyl-2-pyrazolin-5-one;
1-benzyl-3-methyl-2-pyrazolin-5-one;
1-(α-naphthyl)-3-methyl-2-pyrazolin-5-one;
1-methyl-3-phenyl-2-pyrazolin-5-one;
3-methyl-1-(4-methylphenyl)-2-pyrazolin-5-one;
1-(4-butylphenyl)-3-methyl-2-pyrazolin-5-one;
1-(4-methoxyphenyl)-3-methyl-2-pyrazolin-5-one;
1-(4-butoxyphenyl)-3-methyl-2-pyrazolin-5-one;
1-(4-chlorophenyl)-3-methyl-2-pyrazolin-5-one;
1-(4-hydroxyphenyl)-3-methyl-2-pyrazolin-5-one;
1-(3,4-dihydroxyphenyl)-3-methyl-2-pyrazolin-5-one;
1-(2-hydroxyphenyl)-3-methyl-2-pyrazolin-5-one;
1-(3-hydroxyphenyl)-3-methyl-2-pyrazolin-5-one;
1-(4-hydroxyphenyl)-3-methyl-2-pyrazolin-5-one;
1-(3,4-hydroxyphenyl)-3-methyl-2-pyrazolin-5-one;
1-(4-hydroxyphenyl)-3-phenyl-2-pyrazolin-5-one;
1-(4-hydroxymethylphenyl)-3-methyl-2-pyrazolin-5-one;
1-(4-aminophenyl)-3-methyl-2-pyrazolin-5-one;
1-(4-methylaminophenyl)-3-methyl-2-pyrazolin-5-one;
1-(4-ethylaminophenyl)-3-methyl-2-pyrazolin-5-one;
1-(4-butylaminophenyl)-3-methyl-2-pyrazolin-5-one;
1-(4-dimethylaminophenyl)-3-methyl-2-pyrazolin-5-one;
1-(acetamidophenyl)-3-methyl-2-pyrazolin-5-one; and
1-(4-cyanophenyl)-3-methyl-2-pyrazolin-5-one:

As an active ingredient of the medicament of the present invention, a compound in a free form represented by the formula (I) as well as a physiologically acceptable salt thereof may also be used. Examples of the physiologically acceptable salt include a salt with mineral acid such as hydrochloric acid, sulfuric acid, hydrogen bromide salt or phosphoric acid; a salt with organic acid such as methanesulfonic acid, p-toluenesulfonic acid, benzenesulfonic acid, acetic acid, glycolic acid, glucuronic acid, maleic acid, fumaric acid, oxalic acid, ascorbic acid, citric acid, salicylic acid, nicotinic acid or tartaric acid; a salt with alkaline metal such as sodium and potassium; a salt with alkaline earth metal such as magnesium or calcium; and a salt with amine such as ammonia, tris(hydroxymethyl)aminomethane, N,N-bis(hydroxyethyl)piperazine, 2-amino-2-methyl-1-propanol, ethanolamine, N-methyl glutamine or L-glutamine. Moreover, a salt with amino acid such as glycine may also be used

As an active ingredient of the medicament of the present invention, a hydrate of a compound represented by the above formula (I) or a physiologically acceptable salt thereof, or a solvate of a compound represented by the above formula (I) or a physiologically acceptable salt thereof, may also be used. The type of an organic solvent used to form a solvate is not specifically limited. For example, methanol, ethanol, ether, dioxane or tetrahydrofuran can be exemplified. Furthermore, the compound represented by the above formula (I) may have 1 or more asymmetric carbons depending on the type of a substituent. A stereoisomer such as an optical isomer or a diastereoisomer may be present. As an active ingredient of the medicament of the present invention, a stereoisomer in a pure form, any mixture of stereoisomers, raceme or the like may also be used.

All the compounds represented by the formula (I) are known, and can be easily synthesized by a person skilled in the art using a method described in, for example, JP Patent Publication (Kokoku) No. 5-31523 B (1993).

The dose of the medicament of the present invention is not specifically limited. In general, the dose, as the weight of a compound (active ingredient) represented by the formula (I), is generally, in the case of oral administration, 0.1 to 1000 mg/kg body weight per day, preferably 0.5 to 50 mg/kg body weight per day, and in the case of parenteral administration, 0.01 to 100 mg/kg body weight per day and preferably 0.1 to 10 mg/kg body weight. Preferably, the above dose is administered once a day or administered on several (2 to 3) different occasions per day, and may be appropriately increased or decreased depending on age, pathological conditions or symptoms.

As the medicament of the present invention, the compound represented by the above formula (I) or the physiologically acceptable salt thereof, or the hydrate or solvate thereof may be administered as it is. In general, it is preferred that a pharmaceutical composition comprising the above substance which is an active ingredient, and a pharmacologically and pharmaceutically acceptable additive, is prepared and administered.

Examples of pharmacologically and pharmaceutically acceptable additives that can be used herein include excipients, disintegrating agents or disintegrating adjuvant agents, binders, lubricants, coating agents, dye, diluents, base, solubilizing agents or solubilizing adjuvant agents, isotonizing agents, pH regulators, stabilizers, propellants and adhesives.

For a pharmaceutical composition appropriate for oral administration, as additives, there can be used for example excipients such as glucose, lactose, D-mannitol, starch or crystalline cellulose; disintegrating agents or disintegrating adjuvant agents such as carboxymethylcellulose, starch or carboxymetylcellulose calcium; binders such as hydroxypropylcellulose, hydroxypropylmethylcellulose, polyvinylpyrrolidone or gelatine; lubricants such as magnesium stearate or talc; coating agents such as hydroxypropylmethylcellulose, saccharose, polyethylene glycol or titanium oxide; and bases such as petrolatum, liquid paraffin, polyethylene glycol, gelatine, kaolin, glycerin, purified water or hard fat.

For a pharmaceutical composition appropriate for injection or drip, there can be used additives, for example, solubilizing agents or solubilizing adjuvant agents such as distilled water for injection, physiological saline or propylene glycol that can constitute an aqueous injection or an injection that is dissolved when used; isotonizing agents such as glucose, sodium chloride, D-mannitol or glycerine; and pH regulators such as inorganic acid, organic acid, inorganic base and organic base.

The form of the medicament of the present invention is not specifically limited, and may be any of the various forms that can be applied by a person skilled in the art. As a medicament appropriate for oral administration, for example, tablets, powders, granules, hard gelatin capsule agents, suppositories or troches can be prepared by using solid pharmaceutical additives, and for example, syrups, emulsions or soft gelatin capsule agents can be prepared by using liquid pharmaceutical additives. Furthermore, as a medicament appropriate for parenteral administration, injections, drops, inhalants, suppositories, percutaneous absorbents, trans-mucosal absorbents or the like can be prepared. A protective agent for the brain (drops) comprising as an active ingredient a compound represented by the above formula (I) has already been clinically used (under the general name "edaravone" and the commercial name "Radicut": produced and marketed by Mitsubishi Pharma Corporation). This commercially available pharmaceutical preparation can be used as it is for the medicament of the present invention.

The medicament of the present invention is effective for inflammatory bowel disease. That is to say, the medicament of the present invention has an action as a preventive agent for preventing inflammatory bowel disease, and/or an action as a therapeutic agent for recovering the inflammatory bowel disease to a normal condition. In addition, since the medicament of the present invention significantly reduces the area of the mucosa injured by colitis in a dextran sulfate sodium-induced large intestine that is a human ulcerative colitis model, it can be used as an intestinal mucosa protective agent. Moreover, since the medicament of the present invention can inhibit myeloperoxidase activity that is an indicator of inflammation in the large intestinal mucosa, it can be used as an agent for inhibiting such myeloperoxidase activity. Furthermore, since myeloperoxidase has been known as a marker of neutrophilic leucocyte activation (Med Sci Sports Exerc. 2003 Feb; 35(2): 348-55), the medicament of the present invention which inhibits the activation of myeloperoxidase can be used as an agent for inhibiting the activation of neutrophilic leucocytes.

In the present specification, the term "inflammatory bowel disease" is used to mean a disease of unknown cause, wherein inflammation occurs in cells in the surface layer of the alimentary canal mucosa of the large intestine, small intestine, etc., the mucosa is thereby lost, and as a result, ulcers or erosions are developed. Specific examples of such inflammatory bowel disease may include ulcerative colitis and Crohn's disease. In the present specification, typical examples of the ulcerative colitis may include intractable ulcerative colitis and fulminant ulcerative colitis.

Moreover, in the present specification, the inhibition of the activation of neutrophilic leucocytes includes inhibition of the infiltration of neutrophilic leucocytes.

The administration route of the medicament of the present invention is not particularly limited, and the medicament can be administered orally or parenterally. For example, the medicament of the present invention can be orally administered for a preventive purpose before therapy of the aforementioned diseases. Alternatively, it can be administered for a preventive purpose via a parenteral route such as an injection or drop, during, before or after surgery. In addition, it can also be administered in the form of an intravenous or arterial injection to patients suffering from the aforementioned disease for the purpose of preventing deterioration of the symptom or alleviating the symptom.

### Examples

The present invention will be described more specifically by the following examples. The scope of the present invention is not limited by the following examples.

### Synthetic Example: Synthesis of 3-methyl-1-phenyl-2-pyrazolin-5-on (hereinafter referred to as edaravone)

13.0 g of ethyl acetoacetate and 10.8 g of phenylhydrazine were added in 50 ml of ethanol, followed by 3 hours of reflux and stirring. After the reaction solution was allowed to stand to cool, the precipitated crystal was collected by filtration, and then recrystalized with ethanol, thereby obtaining 11.3 g of the subject compound in the form of colorless crystals.
Yield: 67%
Melting point: 127.5 to 128.5°C

### Example 1

### (1) Experimental methods

### 1. Materials

The edaravone as synthesized above was dissolved in a small amount of 1 N sodium hydroxide solution. Thereafter, the pH thereof was adjusted to be around neutral.

### 2. Production method of dextran sulfate sodium (DSS)-induced colitis

6-week-old Sprague-Dawley male rats (Clea Japan, Inc.) were used. According to the method described in the previous report (Araki Y. et al., Scand J Gastroentero, 35, 1060-1067, 2000), colitis was induced to develop in the rats as follows. That is to say, 4% (w/w) dextran sulfate sodium (DSS; molecular weight: 5,000; Wako Pure Chemical Industries, Ltd., Osaka) was added to a powder diet MF (Oriental Yeast Co., Ltd.). The rats were fed with the thus obtained mixed diet at an amount of 30 g/day/rat for 8 days. With regard to edaravone administration groups, from immediately after initiation of the administration of DSS, edaravone was subcutaneously injected into the back of each rat, at a dose of 1 mg/kg/day, 5 mg/kg/day, or 20 mg/kg/day, which was dividedly administered in the morning and evening. With regard to a control group, a normal saline solution was subcutaneously injected into the back of each rat in the same above manner.

### 3. Method for evaluating colitis by naked eyes

8 days later, the rats were subjected to anatomy. The blood was collected from the heart chamber. Thereafter, the large intestine (the colon and the rectum) was excised, and the wet weight of the organ was measured. The degree of the injured large intestinal mucosa was evaluated by naked eyes according to the method described in the previous report (Araki Y. et al., Scand J Gastroentero, 35, 1060-1067, 2000). That is, the areas of portions, on which a change in the color tone of the mucosa (redness), an attachment of blood clots, a collapse of mucosal wall area, edema, or erosion were observed, were measured.

### 4. Histological method for evaluating colitis

The mucosa located at 5 mm from the anus side was fixed with a Camoy's fluid for 3 hours, and was then embedded in paraffin. Thereafter, a tissue fragment with a thickness of 5 µm was prepared, followed by hematoxylin-eosin staining. Scoring of colitis was conducted in accordance with the method described in the previous report (Araki Y. et al., Scand J Gastroentero, 35, 1060-1067, 2000). Namely, the degree and size of each of the following 3 items: (i) the absence of the mucosal epithelium, (ii) deformation of crypts, and (iii) the level of infiltration of inflammatory cells, are expressed in the following point system: 0: none; 1: slight and localized; 2: moderate and localized; 3: moderate and broad; and 4: severe and broad. The total points regarding the above 3 items were summed up, and the obtained value was defined as the histological colitis score (damage score) of a rat.

### 5. Myeloperoxidase activity in large intestinal mucosa

Myeloperoxidase activity used as an indicator of inflammation in the large intestinal mucosa was measured by the method described in the previous report (Araki Y. et al., Clin Exp Immunol, 119, 264-269, 2000). Namely, the mucosa located at 10 mm from the anus side was homogenized. The activity of the obtained homogenate was observed as a change in the absorbance at 460 nm obtained as a result of the reaction of H₂O₂ with o-dianisidin.

### 6. Concentrations of malondialdehyde (MDA) and 4-hydroxy-2(E)-nonenal (4-HNE) in large intestinal mucosa

Hyperoxidation of lipids is an important mechanism regarding tissue injury. MDA and 4-HNE are generated as a result of the hyperoxidation of polyunsaturated fatty acids in tissues. Accordingly, the concentrations of MDA and 4-HNE in the large intestinal mucosa were measured, and the obtained values were used as indicators of the state of reactive oxygen species (ROS) in tissues (Matsumura N. et al., Pancreas, 22, 53-7, 2001). That is, the large intestinal mucosa was homogenized with 9 times its volume of 50 mmol/l Tris-buffer (pH 8.0) containing 0.5% Triton X-100, and the concentrations were measured using an assay kit, Lipid peroxidation assay kit (Calbiochem, La Jolla, CA, U.S.A.). The myeloperoxidase activity and the amount of a protein in the homogenate used for measurement of the concentrations of MDA and 4-HNE were measured using Bio-Rad Protein Assay (Bio-Rad laboratories, CA).

### 7. Method for measuring concentration of interleukin-6 (IL-6) in plasma

The concentration of interleukin-6 in the plasma was measured as an indicator of a systemic inflammatory reaction. That is to say, the blood was centrifuged at 10,000 rpm, and then the concentration of interleukin-6 in the plasma was measured by the ELISA method (Rat IL-6 ELISA KIT, BioSource Europe, S.A., Belgium).

### 8. Statistical processing

The results are shown as mean ± SEM. The comparison between groups was carried out by Fisher's PLSD Test. The comparison between non-parametric data was carried out by Kruskal-Wallis test and Dunn's Procedure As A Multiple Comparison Procedure. The p value that is less than 0.05 was defined as a significant difference.

### (2) Experimental results

### 1. Evaluation of colitis by naked eyes

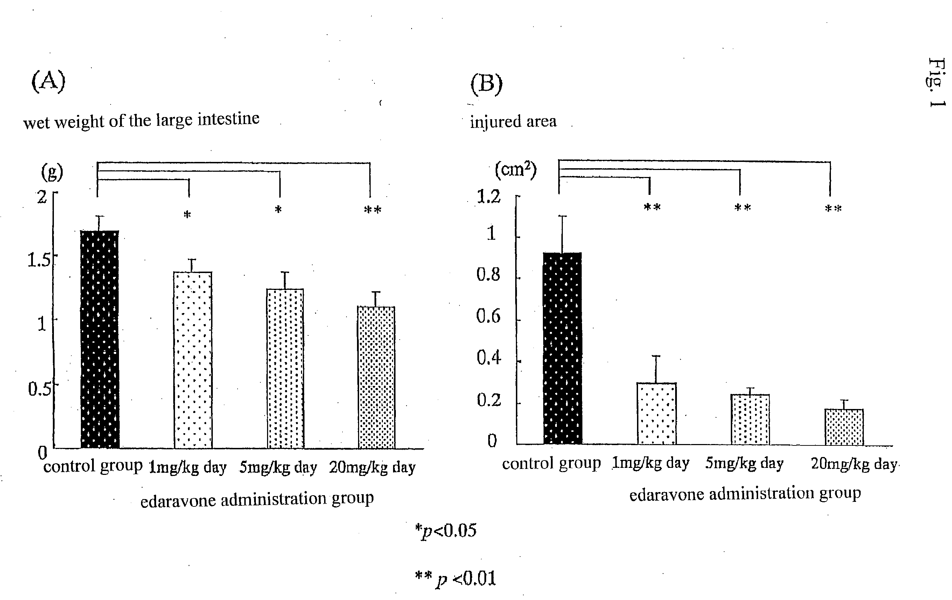

Both in the edaravone administration groups and in the control group, colitis was induced by the administration of DSS for 8 days. However, the wet weight of the large intestine in all of the edaravone administration groups (1 mg/kg administration group, 5 mg/kg administration group, and 20 mg/kg administration group) was significantly lower than that in the control group (Figure 1(A)). Likewise, the injured mucosa area in all of the edaravone administration groups was significantly smaller than that in the control group (Figure 1(B)).

### 2. Histological evaluation of colitis

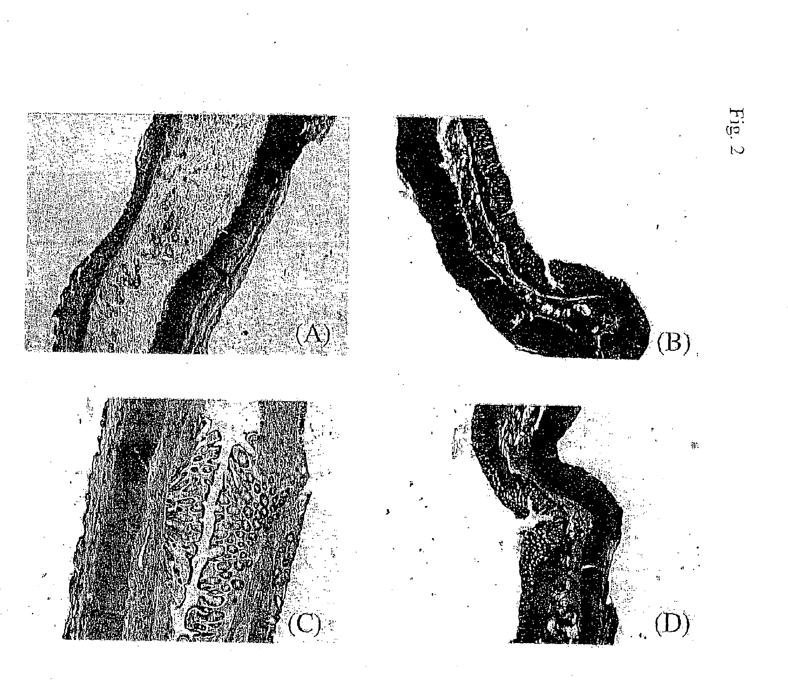

Figure 2 shows the image of the large intestine tissues. (A) is control group (x 40), (B) is 1 mg/kg edaravone administration group (x 40), (C) is 5 mg/kg edaravone administration group (x 40), and (D) is 20 mg/kg edaravone administration group (x 40). In the control group, a significant degree of edema formed in the submucosal layer and infiltration of inflammatory cells were observed. In contrast, in 1 mg/kg, 5 mg/kg, and 20 mg/kg edaravone administration groups, such changes were suppressed.

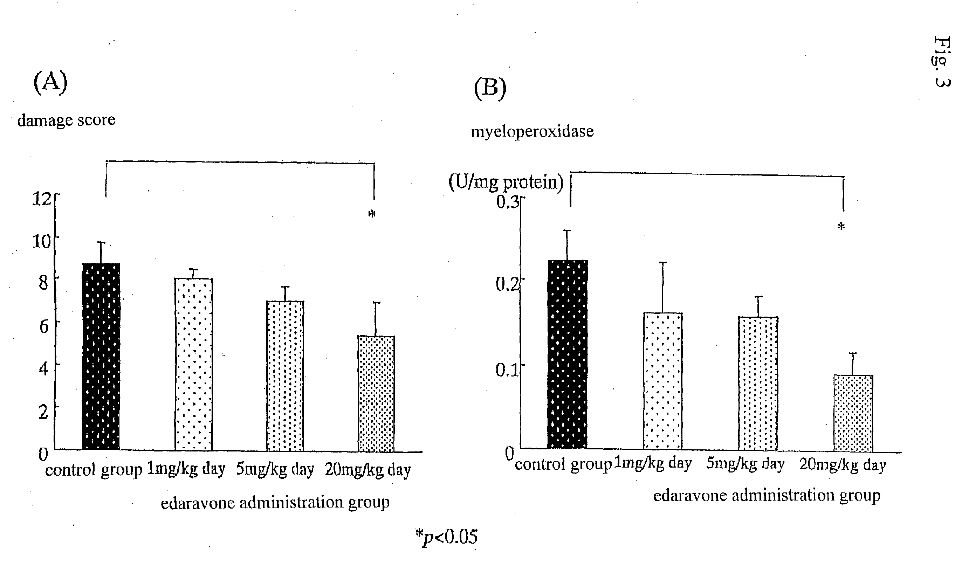

When the degree of damage was expressed with scores, the damage scores in the edaravone administration groups were all lower than that in the control group. In particular, the damage score in the 20 mg/kg edaravone administration group was significantly lower than that in the control group (Figure 3(A)).

### 3. Myeloperoxidase activity in large intestinal mucosa

The myeloperoxidase activity in the large intestinal mucosa in all of the edaravone administration groups was lower than that in the control group. In particular, the myeloperoxidase activity in the 20 mg/kg edaravone administration group was significantly lower than that in the control group (Figure 3(B)).

### 4. Concentrations of MDA and 4-HNE in large intestinal mucosa

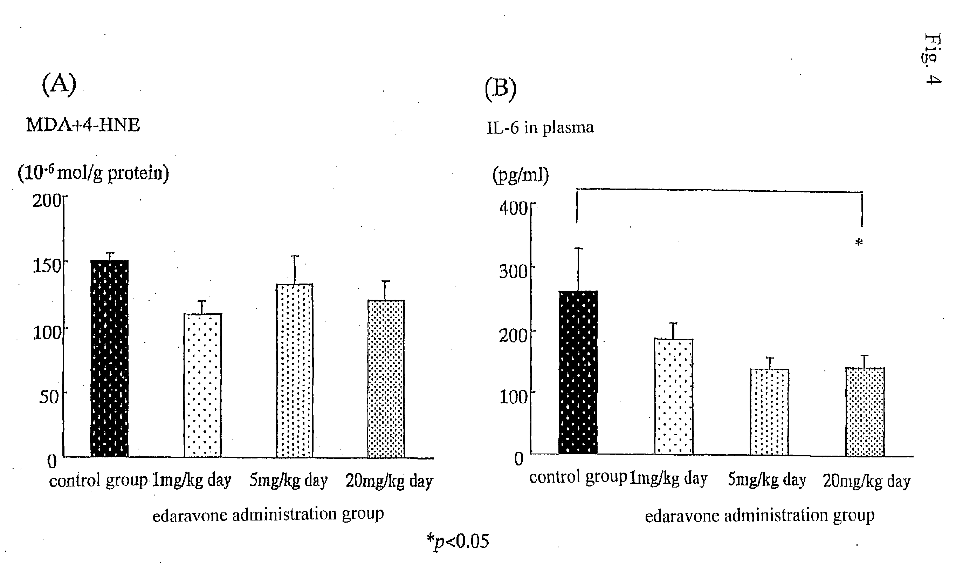

The concentrations of MDA and 4-HNE in the large intestinal mucosa in all of the edaravone administration groups were lower than those in the control group. However, no significant differences were observed among the administration groups (Figure 4(A)).

### 5. Concentration of IL-6 in plasma

The concentration of IL-6 in the plasma in all of the edaravone administration groups was lower than that in the control group. In particular, the concentration of IL-6 in the 20 mg/kg edaravone administration group was significantly lower than that in the control group (Figure 4(B)).

### Industrial Applicability

The medicament of the present invention is useful for preventing and/or treating inflammatory bowel disease. The medicament of the present invention significantly reduces the area of the mucosa injured by colitis in a dextran sulfate sodium-induced large intestine that is a human ulcerative colitis model. Accordingly, the medicament of the present invention is particularly useful as a novel therapeutic agent for patients with ulcerative colitis, and especially for patients suffering from intractable or fulminant ulcerative colitis, which does not react to existing agents.

The entire content of Japanese Patent Application No.2002-262324, which the present application claims a priority based on, is incorporated herein by reference as a part of disclosure of the present specification.

## Claims

1. A medicament for preventing and/or treating inflammatory bowel disease which comprises as an active ingredient a pyrazolone derivative represented by the following formula (I) or a physiologically acceptable salt thereof, or a hydrate thereof or a solvate thereof: 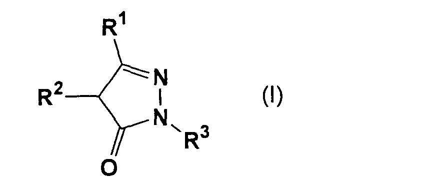 wherein R¹ represents a hydrogen atom, an aryl group, a C₁₋₅ alkyl group, or a C₃₋₆ (total carbon number) alkoxycarbonylalkyl group; R² represents a hydrogen atom, an aryloxy group, an arylmercapto group, a C₁₋₅ alkyl group or a C₁₋₃ hydroxyalkyl group; or R¹ and R² are combined with each other to represent C₃₋₅ alkylene group; and R³ represents a hydrogen atom, a C₁₋₅ alkyl group, a C₅₋₇ cycloalkyl group, a C₁₋₃ hydroxyalkyl group, a benzyl group, a naphthyl group, a phenyl group, or a phenyl group substituted with the same or different 1 to 3 substituents selected from the group consisting of a C₁₋₅ alkyl group, a C₁₋₅ alkoxy group, a C₁₋₃ hydroxyalkyl group, a C₂₋₅ (total carbon number) alkoxycarbonyl group, a C₁₋₃ alkylmercapto group, a C₁₋₄ alkylamino group, a C₂₋₈ (total carbon number) dialkylamino group, a halogen atom, a trifluoromethyl group, a carboxyl group, a cyano group, a hydroxyl group, a nitro group, an amino group and an acetamide group.

2. The medicament according to claim 1 wherein the pyrazolone derivative represented by the formula (I) is 3-methyl-1-phenyl-2-pyrazolin-5-one.

3. The medicament according to claim 1 or 2 wherein the inflammatory bowel disease is ulcerative colitis or Crohn's disease.

4. The medicament according to claim 3 wherein the ulcerative colitis is intractable ulcerative colitis or fulminant ulcerative colitis.

5. An intestinal mucosa protective agent which comprises as an active ingredient a pyrazolone derivative represented by the following formula (I) or a physiologically acceptable salt thereof, or a hydrate thereof or a solvate thereof: 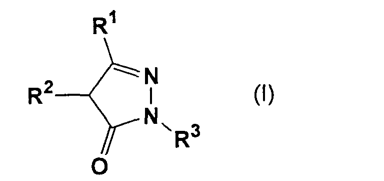 wherein R¹ represents a hydrogen atom, an aryl group, a C₁₋₅ alkyl group, or a C₃₋₆ (total carbon number) alkoxycarbonylalkyl group; R² represents a hydrogen atom, an aryloxy group, an arylmercapto group, a C₁₋₅ alkyl group or a C₁₋₃ hydroxyalkyl group; or R¹ and R² are combined with each other to represent C₃₋₅ alkylene group; and R³ represents a hydrogen atom, a C₁₋₅ alkyl group, a C₅₋₇ cycloalkyl group, a C₁₋₃ hydroxyalkyl group, a benzyl group, a naphthyl group, a phenyl group, or a phenyl group substituted with the same or different 1 to 3 substituents selected from the group consisting of a C₁₋₅ alkyl group, a C₁₋₅ alkoxy group, a C₁₋₃ hydroxyalkyl group, a C₂₋₅ (total carbon number) alkoxycarbonyl group, a C₁₋₃ alkylmercapto group, a C₁₋₄ alkylamino group, a C₂₋₈ (total carbon number) dialkylamino group, a halogen atom, a trifluoromethyl group, a carboxyl group, a cyano group, a hydroxyl group, a nitro group, an amino group and an acetamide group.

6. The intestinal mucosa protective agent according to claim 5 wherein the pyrazolone derivative represented by the formula (I) is 3-methyl-1-phenyl-2-pyrazolin-5-one.

7. An agent for inhibiting the activation of neutrophilic leucocytes which comprises as an active ingredient a pyrazolone derivative represented by the following formula (I) or a physiologically acceptable salt thereof, or a hydrate thereof or a solvate thereof: 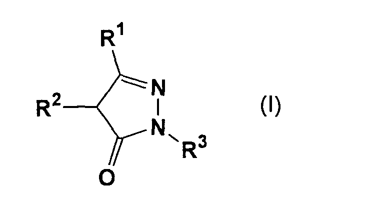 wherein R¹ represents a hydrogen atom, an aryl group, a C₁₋₅ alkyl group, or a C₃₋₆ (total carbon number) alkoxycarbonylalkyl group; R² represents a hydrogen atom, an aryloxy group, an arylmercapto group, a C₁₋₅ alkyl group or a C₁₋₃ hydroxyalkyl group; or R¹ and R² are combined with each other to represent C₃₋₅ alkylene group; and R³ represents a hydrogen atom, a C₁₋₅ alkyl group, a C₅₋₇ cycloalkyl group, a C₁₋₃ hydroxyalkyl group, a benzyl group, a naphthyl group, a phenyl group, or a phenyl group substituted with the same or different 1 to 3 substituents selected from the group consisting of a C₁₋₅ alkyl group, a C₁₋₅ alkoxy group, a C₁₋₃ hydroxyalkyl group, a C₂₋₅ (total carbon number) alkoxycarbonyl group, a C₁₋₃ alkylmercapto group, a C₁₋₄ alkylamino group, a C₂₋₈ (total carbon number) dialkylamino group, a halogen atom, a trifluoromethyl group, a carboxyl group, a cyano group, a hydroxyl group, a nitro group, an amino group and an acetamide group.

8. The agent for inhibiting the activation of neutrophilic leucocytes according to claim 7 wherein the pyrazolone derivative represented by the formula (I) is 3-methyl-1-phenyl-2-pyrazolin-5-one.

9. An agent for inhibiting myeloperoxidase activity which comprises as an active ingredient a pyrazolone derivative represented by the following formula (I) or a physiologically acceptable salt thereof, or a hydrate thereof or a solvate thereof: 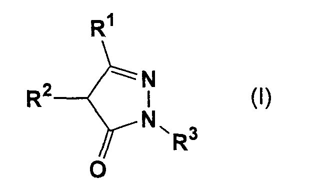 wherein R¹ represents a hydrogen atom, an aryl group, a C₁₋₅ alkyl group, or a C₃₋₆ (total carbon number) alkoxycarbonylalkyl group; R² represents a hydrogen atom, an aryloxy group, an arylmercapto group, a C₁₋₅ alkyl group or a C₁₋₃ hydroxyalkyl group; or R¹ and R² are combined with each other to represent C₃₋₅ alkylene group; and R³ represents a hydrogen atom, a C₁₋₅ alkyl group, a C₅₋₇ cycloalkyl group, a C₁₋₃ hydroxyalkyl group, a benzyl group, a naphthyl group, a phenyl group, or a phenyl group substituted with the same or different 1 to 3 substituents selected from the group consisting of a C₁₋₅ alkyl group, a C₁₋₅ alkoxy group, a C₁₋₃ hydroxyalkyl group, a C₂₋₅ (total carbon number) alkoxycarbonyl group, a C₁₋₃ alkylmercapto group, a C₁₋₄ alkylamino group, a C₂₋₈ (total carbon number) dialkylamino group, a halogen atom, a trifluoromethyl group, a carboxyl group, a cyano group, a hydroxyl group, a nitro group, an amino group and an acetamide group.

10. The agent for inhibiting myeloperoxidase activity according to claim 9 wherein the pyrazolone derivative represented by the formula (I) is 3-methyl-1-phenyl-2-pyrazolin-5-one.

11. The agent for inhibiting myeloperoxidase activity according to claim 9 or 10 wherein the myeloperoxidase is a myeloperoxidase of large intestinal mucosa.

12. A method for preventing and/or treating inflammatory bowel disease which comprises a step of administering to mammals such as a human, a pyrazolone derivative represented by the following formula (I) or a physiologically acceptable salt thereof, or a hydrate thereof or a solvate thereof, at an amount that is effective for prevention and/or treatment of the disease. 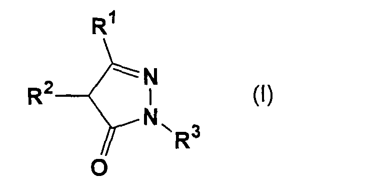 wherein R¹ represents a hydrogen atom, an aryl group, a C₁₋₅ alkyl group, or a C₃₋₆ (total carbon number) alkoxycarbonylalkyl group; R² represents a hydrogen atom, an aryloxy group, an arylmercapto group, a C₁₋₅ alkyl group or a C₁₋₃ hydroxyalkyl group; or R¹ and R² are combined with each other to represent C₃₋₅ alkylene group; and R³ represents a hydrogen atom, a C₁₋₅ alkyl group, a C₅₋₇ cycloalkyl group, a C₁₋₃ hydroxyalkyl group, a benzyl group, a naphthyl group, a phenyl group, or a phenyl group substituted with the same or different 1 to 3 substituents selected from the group consisting of a C₁₋₅ alkyl group, a C₁₋₅ alkoxy group, a C₁₋₃ hydroxyalkyl group, a C₂₋₅ (total carbon number) alkoxycarbonyl group, a C₁₋₃ alkylmercapto group, a C₁₋₄ alkylamino group, a C₂₋₈ (total carbon number) dialkylamino group, a halogen atom, a trifluoromethyl group, a carboxyl group, a cyano group, a hydroxyl group, a nitro group, an amino group and an acetamide group.

13. The method according to claim 12 wherein the pyrazolone derivative represented by the formula (I) is 3-methyl-1-phenyl-2-pyrazolin-5-one.

14. The method according to claim 12 or 13 wherein the inflammatory bowel disease is ulcerative colitis or Crohn's disease.

15. The method according to claim 14 wherein the ulcerative colitis is intractable ulcerative colitis or fulminant ulcerative colitis.

16. A method for protecting the intestinal mucosa which comprises a step of administering to mammals such as a human, a pyrazolone derivative represented by the following formula (I) or a physiologically acceptable salt thereof, or a hydrate thereof or a solvate thereof, at an effective amount. 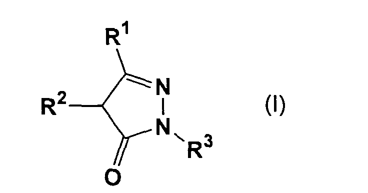 wherein R¹ represents a hydrogen atom, an aryl group, a C₁₋₅ alkyl group, or a C₃₋₆ (total carbon number) alkoxycarbonylalkyl group; R² represents a hydrogen atom, an aryloxy group, an arylmercapto group, a C₁₋₅ alkyl group or a C₁₋₃ hydroxyalkyl group; or R¹ and R² are combined with each other to represent C₃₋₅ alkylene group; and R³ represents a hydrogen atom, a C₁₋₅ alkyl group, a C₅₋₇ cycloalkyl group, a C₁₋₃ hydroxyalkyl group, a benzyl group, a naphthyl group, a phenyl group, or a phenyl group substituted with the same or different 1 to 3 substituents selected from the group consisting of a C₁₋₅ alkyl group, a C₁₋₅ alkoxy group, a C₁₋₃ hydroxyalkyl group, a C₂₋₅ (total carbon number) alkoxycarbonyl group, a C₁₋₃ alkylmercapto group, a C₁₋₄ alkylamino group, a C₂₋₈ (total carbon number) dialkylamino group, a halogen atom, a trifluoromethyl group, a carboxyl group, a cyano group, a hydroxyl group, a nitro group, an amino group and an acetamide group.

17. The method according to claim 16 wherein the pyrazolone derivative represented by the formula (I) is 3-methyl-1-phenyl-2-pyrazolin-5-one.

18. A method for inhibiting the activation of neutrophilic leucocytes which comprises a step of administering to mammals such as a human, a pyrazolone derivative represented by the following formula (I) or a physiologically acceptable salt thereof, or a hydrate thereof or a solvate thereof, at an effective amount. 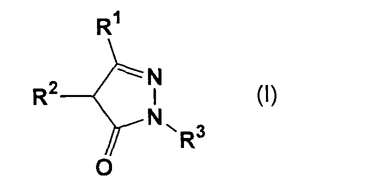 wherein R¹ represents a hydrogen atom, an aryl group, a C₁₋₅ alkyl group, or a C₃₋₆ (total carbon number) alkoxycarbonylalkyl group; R² represents a hydrogen atom, an aryloxy group, an arylmercapto group, a C₁₋₅ alkyl group or a C₁₋₃ hydroxyalkyl group; or R¹ and R² are combined with each other to represent C₃₋₅ alkylene group; and R³ represents a hydrogen atom, a C₁₋₅ alkyl group, a C₅₋₇ cycloalkyl group, a C₁₋₃ hydroxyalkyl group, a benzyl group, a naphthyl group, a phenyl group, or a phenyl group substituted with the same or different 1 to 3 substituents selected from the group consisting of a C₁₋₅ alkyl group, a C₁₋₅ alkoxy group, a C₁₋₃ hydroxyalkyl group, a C₂₋₅ (total carbon number) alkoxycarbonyl group, a C₁₋₃ alkylmercapto group, a C₁₋₄ alkylamino group, a C₂₋₈ (total carbon number) dialkylamino group, a halogen atom, a trifluoromethyl group, a carboxyl group, a cyano group, a hydroxyl group, a nitro group, an amino group and an acetamide group.

19. The method according to claim 18 wherein the pyrazolone derivative represented by the formula (I) is 3-methyl-1-phenyl-2-pyrazolin-5-one.

20. A method for inhibiting myeloperoxidase activity which comprises a step of administering to mammals such as a human, a pyrazolone derivative represented by the following formula (I) or a physiologically acceptable salt thereof, or a hydrate thereof or a solvate thereof, at an effective amount. 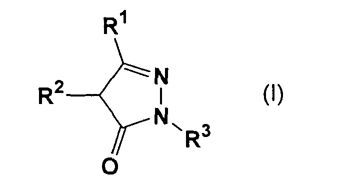 wherein R¹ represents a hydrogen atom, an aryl group, a C₁₋₅ alkyl group, or a C₃₋₆ (total carbon number) alkoxycarbonylalkyl group; R² represents a hydrogen atom, an aryloxy group, an arylmercapto group, a C₁₋₅ alkyl group or a C₁₋₃ hydroxyalkyl group; or R¹ and R² are combined with each other to represent C₃₋₅ alkylene group; and R³ represents a hydrogen atom, a C₁₋₅ alkyl group, a C₅₋₇ cycloalkyl group, a C₁₋₃ hydroxyalkyl group, a benzyl group, a naphthyl group, a phenyl group, or a phenyl group substituted with the same or different 1 to 3 substituents selected from the group consisting of a C₁₋₅ alkyl group, a C₁₋₅ alkoxy group, a C₁₋₃ hydroxyalkyl group, a C₂₋₅ (total carbon number) alkoxycarbonyl group, a C₁₋₃ alkylmercapto group, a C₁₋₄ alkylamino group, a C₂₋₈ (total carbon number) dialkylamino group, a halogen atom, a trifluoromethyl group, a carboxyl group, a cyano group, a hydroxyl group, a nitro group, an amino group and an acetamide group.

21. The method according to claim 20 wherein the pyrazolone derivative represented by the formula (I) is 3-methyl-1-phenyl-2-pyrazolin-5-one.

22. The method according to claim 20 or 21 wherein the myeloperoxidase is a myeloperoxidase of large intestinal mucosa.

23. Use of a pyrazolone derivative represented by following formula (I) or a physiologically acceptable salt thereof, or a hydrate thereof or a solvate thereof, for the production of a medicament for preventing and/or treating inflammatory bowel disease. 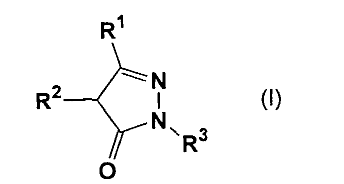 wherein R¹ represents a hydrogen atom, an aryl group, a C₁₋₅ alkyl group, or a C₃₋₆ (total carbon number) alkoxycarbonylalkyl group; R² represents a hydrogen atom, an aryloxy group, an arylmercapto group, a C₁₋₅ alkyl group or a C₁₋₃ hydroxyalkyl group; or R¹ and R² are combined with each other to represent C₃₋₅ alkylene group; and R³ represents a hydrogen atom, a C₁₋₅ alkyl group, a C₅₋₇ cycloalkyl group, a C₁₋₃ hydroxyalkyl group, a benzyl group, a naphthyl group, a phenyl group, or a phenyl group substituted with the same or different 1 to 3 substituents selected from the group consisting of a C₁₋₅ alkyl group, a C₁₋₅ alkoxy group, a C₁₋₃ hydroxyalkyl group, a C₂₋₅ (total carbon number) alkoxycarbonyl group, a C₁₋₃ alkylmercapto group, a C₁₋₄ alkylamino group, a C₂₋₈ (total carbon number) dialkylamino group, a halogen atom, a trifluoromethyl group, a carboxyl group, a cyano group, a hydroxyl group, a nitro group, an amino group and an acetamide group.

24. The use according to claim 23 wherein the pyrazolone derivative represented by the formula (I) is 3-methyl-1-phenyl-2-pyrazolin-5-one.

25. The use according to claim 23 or 24 wherein the inflammatory bowel disease is ulcerative colitis or Crohn's disease.

26. The use according to claim 25 wherein the ulcerative colitis is intractable ulcerative colitis or fulminant ulcerative colitis.

27. Use of a pyrazolone derivative represented by following formula (I) or a physiologically acceptable salt thereof, or a hydrate thereof or a solvate thereof, for the production of an intestinal mucosa protective agent. 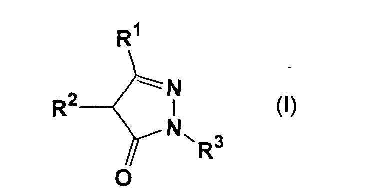 wherein R¹ represents a hydrogen atom, an aryl group, a C₁₋₅ alkyl group, or a C₃₋₆ (total carbon number) alkoxycarbonylalkyl group; R² represents a hydrogen atom, an aryloxy group, an arylmercapto group, a C₁₋₅ alkyl group or a C₁₋₃ hydroxyalkyl group; or R¹ and R² are combined with each other to represent C₃₋₅ alkylene group; and R³ represents a hydrogen atom, a C₁₋₅ alkyl group, a C₅₋₇ cycloalkyl group, a C₁₋₃ hydroxyalkyl group, a benzyl group, a naphthyl group, a phenyl group, or a phenyl group substituted with the same or different 1 to 3 substituents selected from the group consisting of a C₁₋₅ alkyl group, a C₁₋₅ alkoxy group, a C₁₋₃ hydroxyalkyl group, a C₂₋₅ (total carbon number) alkoxycarbonyl group, a C₁₋₃ alkylmercapto group, a C₁₋₄ alkylamino group, a C₂₋₈ (total carbon number) dialkylamino group, a halogen atom, a trifluoromethyl group, a carboxyl group, a cyano group, a hydroxyl group, a nitro group, an amino group and an acetamide group.

28. The use according to claim 27 wherein the pyrazolone derivative represented by the formula (I) is 3-methyl-1-phenyl-2-pyrazolin-5-one.

29. Use of a pyrazolone derivative represented by following formula (I) or a physiologically acceptable salt thereof, or a hydrate thereof or a solvate thereof, for the production of an agent for inhibiting the activation of neutrophilic leucocytes. 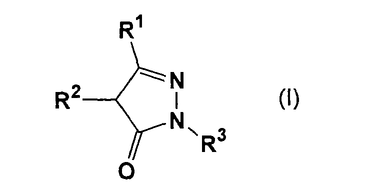 wherein R¹ represents a hydrogen atom, an aryl group, a C₁₋₅ alkyl group, or a C₃₋₆ (total carbon number) alkoxycarbonylalkyl group; R² represents a hydrogen atom, an aryloxy group, an arylmercapto group, a C₁₋₅ alkyl group or a C₁₋₃ hydroxyalkyl group; or R¹ and R² are combined with each other to represent C₃₋₅ alkylene group; and R³ represents a hydrogen atom, a C₁₋₅ alkyl group, a C₅₋₇ cycloalkyl group, a C₁₋₃ hydroxyalkyl group, a benzyl group, a naphthyl group, a phenyl group, or a phenyl group substituted with the same or different 1 to 3 substituents selected from the group consisting of a C₁₋₅ alkyl group, a C₁₋₅ alkoxy group, a C₁₋₃ hydroxyalkyl group, a C₂₋₅ (total carbon number) alkoxycarbonyl group, a C₁₋₃ alkylmercapto group, a C₁₋₄ alkylamino group, a C₂₋₈ (total carbon number) dialkylamino group, a halogen atom, a trifluoromethyl group, a carboxyl group, a cyano group, a hydroxyl group, a nitro group, an amino group and an acetamide group.

30. The use according to claim 29 wherein the pyrazolone derivative represented by the formula (I) is 3-methyl-1-phenyl-2-pyrazolin-5-one.

31. Use of a pyrazolone derivative represented by following formula (I) or a physiologically acceptable salt thereof, or a hydrate thereof or a solvate thereof, for the production of an agent for inhibiting myeloperoxidase activity. 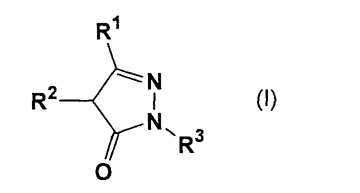 wherein R¹ represents a hydrogen atom, an aryl group, a C₁₋₅ alkyl group, or a C₃₋₆ (total carbon number) alkoxycarbonylalkyl group; R² represents a hydrogen atom, an aryloxy group, an arylmercapto group, a C₁₋₅ alkyl group or a C₁₋₃ hydroxyalkyl group; or R¹ and R² are combined with each other to represent C₃₋₅ alkylene group; and R³ represents a hydrogen atom, a C₁₋₅ alkyl group, a C₅₋₇ cycloalkyl group, a C₁₋₃ hydroxyalkyl group, a benzyl group, a naphthyl group, a phenyl group, or a phenyl group substituted with the same or different 1 to 3 substituents selected from the group consisting of a C₁₋₅ alkyl group, a C₁₋₅ alkoxy group, a C₁₋₃ hydroxyalkyl group, a C₂₋₅ (total carbon number) alkoxycarbonyl group, a C₁₋₃ alkylmercapto group, a C₁₋₄ alkylamino group, a C₂₋₈ (total carbon number) dialkylamino group, a halogen atom, a trifluoromethyl group, a carboxyl group, a cyano group, a hydroxyl group, a nitro group, an amino group and an acetamide group.

32. The use according to claim 31 wherein the pyrazolone derivative represented by the formula (I) is 3-methyl-1-phenyl-2-pyrazolin-5-one.

33. The use according to claim 31 or 32 wherein the myeloperoxidase is a myeloperoxidase of large intestinal mucosa.
